## Europäisches Patentamt

## European Patent Office

## Office européen des brevets

(19)

(11) Veröffentlichungsnummer: **0 252 311**
**B1**

(12) # EUROPÄISCHE PATENTSCHRIFT

(45) Veröffentlichungstag der Patentschrift:
**29.08.90**

(51) Int. Cl.⁵: **G01N 19/04**

(21) Anmeldenummer: **87108242.6**

(22) Anmeldetag: **06.06.87**

(54) Verfahren und Vorrichtung zum Messen von Überzugs-Schichtstärken an Kraftfahrzeug-Karosserien.

(30) Priorität: **05.07.86 DE 3622708**

(43) Veröffentlichungstag der Anmeldung:
**13.01.88 Patentblatt 88/2**

(45) Bekanntmachung des Hinweises auf die Patenterteilung:
**29.08.90 Patentblatt 90/35**

(84) Benannte Vertragsstaaten:
**DE ES FR GB IT SE**

(56) Entgegenhaltungen:
DE-A- 1 956 098
DE-A- 2 336 212
DE-A- 2 742 058
GB-A- 2 166 876

NATIONAL TECHNICAL INFORMATION SERVICE
SELECTED TECHNOLOGY FOR LICENSING, 11.
Februar 1981, Nr. 272231, Seiten 2301-2302

(73) Patentinhaber: **FORD-WERKE AKTIENGESELLSCHAFT,
Werk Köln-Niehl Henry-Ford-Strasse Postfach 60 40 02,
D-5000 Köln 60(DE)**

(84) Benannte Vertragsstaaten: **DE IT SE**

(73) Patentinhaber: **FORD MOTOR COMPANY LIMITED,
Eagle Way, Brentwood Essex CM13 3BW(GB)**

(84) Benannte Vertragsstaaten: **GB**

(73) Patentinhaber: **FORD FRANCE SOCIETE ANONYME,
344 Avenue Napoléon Bonaparte B.P. 307, F-92506 Rueil
Malmaison Cedex(FR)**

(84) Benannte Vertragsstaaten: **FR**

(73) Patentinhaber: **Ford Motor Company, One Parklane
Boulevard Parklane Towers East, Dearborn
Michigan 48126(US)**

(84) Benannte Vertragsstaaten: **ES**

(72) Erfinder: **Lang, Hans, Braunsberger Strasse 20,
D-4000 Düsseldorf 13(DE)**

(74) Vertreter: **Ritzkowsky, Harald, Dipl.-Ing., Ford-Werke
Aktiengesellschaft Patentabteilung NH/DRP
Henry-Ford-Strasse, D-5000 Köln 60(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Verfahren und eine Vorrichtung zum Messen von Überzugs-Schichtstärken an Kraftfahrzeug-Karosserien der im Oberbegriff des Patentanspruches erläuterten Art.

Aus der Zeitschrift "Automobil Revue" Nr. 13 vom 27.03.1986, Seite 51, ist ein Verfahren zum Prüfen von Endlackierungen an Kraftfahrzeug-Karosserien bekannt, bei dem eine Meßsonde über Handhabungsautomaten an einer Karosserie entlanggeführt wird.

Der hierfür erforderliche Handhabungsautomat muß über entsprechende Sensoren feinfühlig gesteuert werden, damit er die Meßsonde entlang der gewölbten Kontur einer Karosserie entlangführen kann. Dies bedingt einen verhältnismäßig aufwendigen Handhabungsautomaten mit einer sehr aufwendigen Sensoren-Steuereinrichtung. Weiterhin muß hierbei die Karosserie während des Meßvorganges stationär gehalten werden, so daß diese Messung nicht unmittelbar im normalen Produktionsablauf einer Karosseriefertigung angeordnet werden kann.

Die Aufgabe der Erfindung ist es ein Verfahren zum Messen von Überzugs-Schichtstärken an Kraftfahrzeug-Karosserien der im Oberbegriff des Patentanspruches 1 erläuterten Art derart zu verbessern, daß die Messung bei sich bewegender Karosserie mittels nur einfachen Handhabungsautomaten ererfolgen kann.

Gemäß der Erfindung wird diese Aufgabe gelöst, indem bei einem Verfahren zum Messen von Überzugs-Schichtstärken an Kraftfahrzeug-Karosserien der im Oberbegriff des Patentanspruches 1 erläuterten Art die im Kennzeichenteil des Patentanspruches 1 aufgezeigten Verfahrensschritte durchgeführt werden.

Im Patentanspruch 2 ist eine Vorrichtung zur Durchführung des Verfahrens nach Patentanspruch 1 in ihren baulichen Ausgestaltungen erläutert.

Dadurch, daß die in einem kardanisch aufgehängtem Führungsteil angeordnete Meßsonde etwa senkrecht zur Oberfläche einer sich bewegenden Karosserie herangeführt wird; mit einer bestimmten Andrückkraft senkrecht auf die Oberfläche der Karosserie aufgesetzt wird, über einen bestimmten Zeitabschnitt festgehalten und mitbewegt wird und danach abgehoben und in ihre Ausgangslage zurückgeführt wird, kann eine aufwendige Synchronisation zwischen der Bewegung des Handhabungsautomaten und der Bewegung der Karosserie entfallen.

Dadurch, daß ein Handhabungsautomat eine Meßsondenanordnung entlang dreier Achsen X, Y und Z bewegbar aufnimmt, die Meßsondenanordnung in einem kardanisch aufgehängten Führungsteil angeordnet ist und in Z-Richtung etwa senkrecht zur Oberfläche einer sich bewegenden Karosserie herangeführt und über eine im Führungsteil entgegen einer Feder axial verschiebbaren Hülsenteil und einem daran befestigten Saugnapf mit innerem Anschlagring weich aufgesetzt wird und sich die Meßsondenanordnung durch den inneren Anschlagring senkrecht zur Oberfläche der Karosserie ausrichtet und durch Beaufschlagung des Saugnapfes mit Unterdruck an der Oberfläche der Karosserie festgehalten wird, wobei die im Hülsenteil über eine Feder axial verschiebbare Meßsonde mit einer vorbestimmten Andrückkraft auf die Oberfläche der Karosserie gedrückt wird, wird mit einfachem Bauaufwand eine zuverläßige Messung der Überzugs-Schichtstärke entlang einer Linie vorbestimmter Meßpunkte erzielt, wobei die sich bewegende Karosserie keinen Einfluß auf die Genauigkeit der Messungen ausübt.

Aus der DE-OS 27 42 058 ist zwar bereits eine Vorrichtung zum Anbringen von Meßwertaufnehmen und Meßelektroden an Oberflächen bekannt, die aus einem die Meßsonde umschließenden Saugnapf besteht, der mit Unterdruck beaufschlagbar ist, dieser Saugnapf mit Meßsonde ist jedoch zum Aufsetzen auf Hautoberflächen vorgesehen und weist keine Einrichtungen zum senkrechten Ausrichten und Andrücken der Meßsonde mit einer bestimmten Andrückkraft auf.

Die Erfindung wird anhand eines in der beiliegenden Zeichnung gezeigten Ausführungsbeispieles näher erläutert. Es zeigt:

Figur 1 eine schematische Frontansicht auf eine Vorrichtung gemäß der Erfindung;

Figur 2 eine schematische Draufsicht auf eine Vorrichtung gemäß der Erfindung und

Figur 3 einen vertikalen Schnitt durch die an der Z-Achse des Handhabungsautomaten angeordnete erfindungsgemäße Meßsondenanordnung.

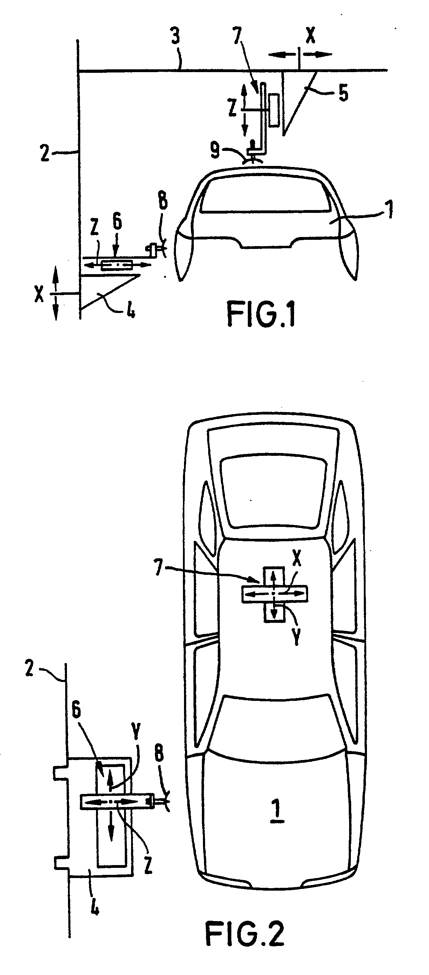

In den Figuren 1 und 2 ist die Karosserie 1 eines Kraftfahrzeuges schematisch dargestellt, die über eine an sich bekannte, nicht gezeigte Fördereinrichtung in Längsrichtung bewegt wird. Benachbart diesem Bewegungsweg sind an einer Seitenwand 2 bzw. an einer Decke 3 Konsolen 4 bzw. 5 angeordnet auf denen einfache Handhabungsautomaten 6 bzw. 7 angeordnet sind, die in X-Richtung, das heißt, senkrecht bzw. quer zur Bewegungsrichtung der Karosserie 1, in Y-Richtung, das heißt, parallel zur Bewegungsrichtung der Karosserie 1 und in Z-Richtung, das heißt, senkrecht zur Längsmittelebene bzw. senkrecht zur Bodenfläche der Karosserie 1 bewegbare Elemente besitzen.

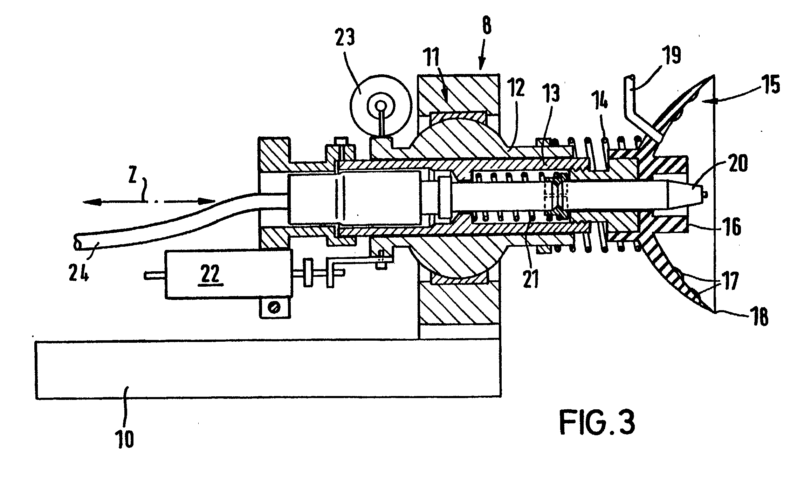

An den in Z-Richtung bewegbaren Elementen der Handhabungsautomaten 6 bzw. 7 sind Meßsondenanordnungen 8 bzw. 9 angeordnet, deren Aufbau im Zusammenhang mit Figur 3 näher erläutert wird.

Figur 3 zeigt einen vertikalen Schnitt durch die Z-Achse eines Handhabungsautomaten, von dem nur die Bewegungseinrichtung 10 für die Z-Achse angedeutet ist. An der Bewegungseinrichtung 10 ist eine Meßsondenanordnung 8 befestigt.

Die Meßsondenanordnung 8 besteht im wesentlichen aus einem in einem Kugelgelenk 11 kardanisch aufgehängtem Führungsteil 12 in dem ein Hülsenteil 13 entgegen der Kraft einer Feder 14 axial verschiebbar angeordnet ist. Das Hülsenteil 13 trägt an seinem vorderen Ende einen Saugnapf 15 mit einem inneren Anschlagring 16, Anschlagwarzen 17 und einer äußeren Sauglippe 18. Das nnere des Saugnapfes 15 kann über eine Unterdruckleitung 19 mit Unterdruck beaufschlagt werden.

Innerhalb des Saugnapfes 15 und innerhalb des inneren Anschlagringes 16 ist eine Meßsonde 20 entgegen der Kraft einer Feder 21 axial verschiebbar im Hülsenteil 13 angeordnet.

An dem Führungsteil 12 greifen über entsprechende Gestänge zwei Wegesensoren 22 und 23 an.

Die durch die Meßsonde 20 erfaßten Meßwerte werden über ein Sondenkabel 24 einer Auswert- oder Anzeigeeinrichtung zugeleitet.

Zu Beginn einer Messung wird die Meßsondenanordnung 8 über die Bewegungseinrichtung 10 schnell etwa senkrecht zur Oberfläche der Karosserie bewegt. Sobald der Saugnapf 15 auf die Oberfläche der Karosserie (nicht gezeigt) auftrifft stützt er sich über die Anschlagwarzen 17 ab und die Meßsondenanordnung 8 richtet sich durch die kardanische Aufhängung über das Kugelgelenk 11 senkrecht zur Oberfläche der Karosserie aus. Sobald die Meßsondenanordnung 8 auf die Oberfläche einer Karosserie auftrifft, wird die entsprechende Wegbegrenzung in der Z-Achse durch den Wegsensor 22 erfaßt und schaltet die Bewegungseinrichtung 10 ab. Die Anlage des Saugnapfes 15 an der Oberfläche der Karosserie führt jedoch bei der sich bewegenden Karosserie zu einer Mitnahme des Saugnapfes 15 und diese Bewegung in Richtung der Achse Y wird durch den Wegsensor 23 erfaßt und schaltet die Bewegungseinrichtung des Handhabungsautomaten in Richtung der Y-Achse ab und veranlaßt die Beaufschlagung des Unterdruckanschluß 19 mit Unterdruck, wodurch der Saugnapf 15 nunmehr so eng an die Oberfläche der Karosserie herangezogen wird, daß er die gesamte Meßsondenanordnung 8 unverrückbar an der sich bewegenden Karosserie festhält und mitnimmt.

Durch das Ansaugen des Saugnapfes 15 an der Oberfläche der Karosserie wird der innere Anschlagring 16 angelegt und die innerhalb des inneren Anschlagringes 16 angeordnete Meßsonde 20 wird entgegen der Kraft ihrer Feder 21 axial zurückgedrückt und stellt hierdurch den für eine genaue Messung erwünschten gleichbleibenden Anpreßdruck sicher. Sobald das Meßergebnis über das Sondenkabel 24 der Meß- oder Anzeigeeinrichtung mitgeteilt wurde, wird von dieser aus die Unterdruckbeaufschlagung des Saugnapfes 15 gelöst, wodurch der Saugnapf 15 von der Oberfläche der Karosserie abhebt und die Wegesensoren 22 und 23 wieder die Bewegungseinrichtungen des Handhabungsautomaten aktivieren, die die Meßsondenanordnung 8 zunächst von der Karosserie abrücken und dann entgegen der Bewegungsrichtung der Karosserie wieder in ihre Ausgangslage zurückführen.

Dadurch, daß die Meßsonde während des Meßvorganges über den Saugnapf unverrückbar an der Oberfläche der Karosserie festgehalten wird, kann während dieses Zeitabschnittes eine aufwendige Synchronisierung zwischen der Bewegung der Karosserie und einer Bewegung des Handhabungsautomaten entfallen, wodurch der bauliche Aufwand für diesen wesentlich einfacher gehalten werden kann. Die Bewegungen die mit dem Handhabungsautomaten ausgeführt werden müssen, umfassen nur das schnelle Heranführen der Meßsondenanordnung an die Oberfläche der Karosserie, während nach dem Anhaften des Saugnapfes die Bewegungseinrichtungen des Handhabungsautomaten ausgelöst von den den Bewegungssensoren für die Z- und Y-Richtung freilaufend gehalten werden und erst nach dem Abheben der Meßsondenanordnung wieder betätigt werden um die Meßsondenanordnung von der Karosserie weg und in ihre Ausgangslage zurückzuführen.

Durch das Verfahren und die Vorrichtung gemäss der Erfindung ist ein einwandfreies Messen der Überzugs-Schichtstärke an Kraftfahrzeug-Karosserien während des Bewegungsablaufes einer Karosserie möglich, so daß entsprechende Meßeinrichtungen bei Bedarf unmittelbar an den Stellen der Produktionslinie angeordnet werden können, an denen die Karosserie aus einer der vielen Überzugs-Auftragsbereiche im Oberflächenbehandlungsbereich herausgeführt werden.

**Patentansprüche**

1. Verfahren zum Prüfen von Endlackierungen an Kraftfahrzeug-Karosserien mittels über Handhabungsautomaten an einer Karosserie (1) entlanggeführten Meßsonden, gekennzeichnet durch folgende Verfahrensschritte:

a) Heranführen einer in einem kardanisch aufgehängten Führungsteil (12) angeordneten Meßsonde (20) etwa senkrecht zur Oberfläche der sich bewegenden Karosserie (1);

b) Aufsetzen der Meßsonde (20) senkrecht zur Oberfläche der Karosserie (1) mit einer bestimmten Andrückkraft;

c) Mitbewegen der Meßsonde (20) mit der Karosserie (1) über einen bestimmten Zeitabschnitt und

d) Abheben und Zurückführen der Meßsonde (20) in ihre Ausgangslage.

2. Vorrichtung zur Durchführung des Verfahrens nach Anspruch 1, **dadurch gekennzeichnet**, daß ein Handhabungsautomat (6 bzw. 7) eine Meßsondenanordnung (8 bzw. 9) entlang dreier Achsen X, Y und Z bewegbar aufnimmt, wobei die Meßsondenanordnung (8) in Z-Richtung über eine Bewegungseinrichtung (10) etwa senkrecht zur Oberfläche der Karosserie (1) herangeführt wird und in einem kardanisch aufgehängten Führungsteil (12) ein Hülsenteil (13) entgegen der Kraft einer Feder (14) axial verschiebbar ist, der an seinem vorderen Ende einen Saugnapf (15) mit einem inneren Anschlagring (16) Anschlagwarzen (17) und einer äußeren Sauglippe (18) trägt und innerhalb des Saugnapfes (15) eine Meßsonde (20) entgegen der Kraft einer Feder (21) axial verschiebbar angeordnet ist und der Saugnapf (15) über eine Unterdruckleitung (19) mit Unterdruck beaufschlagbar ist und wobei Bewegungsreaktionen des Führungsteiles (12) über Wegesensoren (22 und 23) die Bewegungseinrichtungen des Handhabungsautomaten bei angehaftetem Saugnapf (15) freilaufend schalten.

## Claims

1. A method of testing final lacquering layer thicknesses on motor vehicle bodies by means of measuring probes moved along a body (1) by way of automatic manipulators, characterized by the following method steps:

a) moving a measuring probe (20) disposed in a guide portion (12) suspended on gimbals approximately at right angles to the surface of the moving body (1);

b) placing the measuring probe (20) vertically upon the surface of the body (1) with a specific pressure;

c) jointly moving the measuring probe (20) with the body (1) for a specific period of time:

d) raising and moving the measuring probe (20) back to its initial position.

2. An apparatus for performing the method according to Claim 1, characterized in that an automatic manipulator (6 and 7 respectively) receives a measuring probe arrangement (8 and 9 respectively) so as to be movable along three axes X, Y and Z, the measuring probe arrangement (8) being guided in the Z-direction approximately at right angles to the surface of the body (1) by way of a displacement device (10) and in a guide portion (12) suspended on gimbals a sleeve portion (13) is axially displaceable against the force of a spring (14) and at its front end is provided with a suction cup (15) with an inner stop ring (16), stop pegs (17) and an outer suction lip (18) and inside the suction cup (15) a measuring probe (20) is arranged so as to be axially displaceable against the force of a spring (21) and the suction cup (15) can be acted upon with underpressure by way of an underpressure line (19), movement reactions of the guide portion (12) actuating the displacement devices of the automatic manipulators by way of path sensors (22 and 23) in a freely running manner when the suction cup is attached.

## Revendications

1. Procédé de vérification des peintures de finition de carrosseries de véhicules automobiles, à l'aide de sondes de mesure guidées par des manipulateurs automatiques le long d'une carrosserie (1), caractérisé par les étapes de procédé suivantes:

a) approche d'une sonde de mesure (20) placée dans un élément de guidage (12) suspendu par un joint de cardan, à peu près perpendiculairement à la surface de la carrosserie (1) en mouvement;

b) mise en place de la sonde de mesure (20) perpendiculairement à la surface de la carrosserie (1) avec une force de pression définie;

c) déplacement de la sonde de mesure (20) avec la carrosserie (1) pendant un intervalle de temps déterminé, et

d) relevage et retour de la sonde de mesure (20) dans sa position initiale.

2. Dispositif de mise en œuvre du procédé selon la revendication 1, caractérisé en ce qu'un manipulateur automatique (6 ou 7) loge un dispositif à sonde de mesure (8 ou 9) pour le déplacer le long de trois axes X, Y et Z, le dispositif à sonde de mesure (8) étant approché à peu près perpendiculairement à la surface de la carrosserie (1), dans la direction Z, par un dispositif de déplacement (10), et en ce que dans un élément de guidage (12) suspendu par un joint de cardan, un élément formant fourreau (13) peut coulisser axialement à l'encontre de la force d'un ressort (14), lequel élément formant fourreau porte à son extrémité avant une ventouse (15) avec une bague de butée intérieure (16), des boutons de butée (17) et une lèvre d'aspiration (18), en ce qu'à l'intérieur de la ventouse (15) est placée une sonde de mesure (20) qui coulisse axialement à l'encontre de la force d'un ressort (21), en ce que la ventouse (15) est soumise à une dépression par un conduit de dépression (9), et en ce que des réactions de déplacement de l'élément de guidage (12) commandent en course libre les dispositifs de déplacement du manipulateur automatique, avec ventouse (15) adhérente, par des capteurs de parcours (22 et 23).

FIG.1

FIG.2

FIG.3

EP 0 252 311 B1